# EUROPEAN PATENT SPECIFICATION

(11) **EP 1 975 145 B1**
(45) Date of publication and mention of the grant of the patent: **19.06.2013**
(21) Application number: 06832838.4
(22) Date of filing: 17.11.2006
(51) Int. Cl.: C07C 45/33, C07C 27/26, C07C 29/88, C07C 45/85, C07C 49/403

(54) **METHOD OF RECOVERING CYCLOHEXANONE AND CYCLOHEXANOL**
VERFAHREN ZUR RÜCKGEWINNUNG VON CYCLOHEXANON UND CYCLOHEXANOL
PROCEDE DE RECUPERATION DE CYCLOHEXANONE ET DE CYCLOHEXANOL

(30) Priority: 06.12.2005 JP 2005351446
(43) Date of publication of application: 01.10.2008
(73) Proprietor: Ube Industries, Ltd., Ube-shi Yamaguchi 755-8633 (JP)
(72) Inventor: KUGIMOTO, Junichi, Yamaguchi 755-8633 (JP); KAISO, Kouji, Yamaguchi 755-8633 (JP); SHIMOMURA, Hideo, Yamaguchi 755-8633 (JP)
(74) Representative: Bublak, Wolfgang
(86) International application number: PCT/JP2006/322959
(87) International publication number: WO 2007/066491

(56) References cited:
- JP-A- 08 081 407
- JP-A- 10 182 522
- JP-A- 2004 099 545
- US-B1- 6 255 533

## Description

### TECHNICAL FIELD

The present invention relates to a method of recovering cyclohexanone and cyclohexanol from a high-boiling-point mixture produced as by-product in a process for producing cyclohexanone through a liquid phase oxidation of cyclohexane by an oxygenous gas.

### BACKGROUND ART

In a process for producing cyclohexanone (hereinafter referred to as anone) through a liquid phase oxidation of cyclohexane (hereinafter referred to as Cx) by an oxygenous gas, a substance having a higher boiling point than anone is produced as by-product in each of the following steps as known.
[Step A] The step of liquid-phase oxidizing Cx and the subsequent post-treatment step (such as the step of decomposing peroxides in acidic reaction solutions); hereinafter, a high-boiling-point substance produced in this step is referred to as High-boiling-point substance A.
[Step B] The step of dehydrogenating cyclohexanol (hereinafter referred to as anol) to invert it into anone; hereinafter, a high-boiling-point substance produced in this step is referred to as High-boiling-point substance B.
[Step C] The step of distillation purifying anone and anol; hereinafter, a high-boiling-point substance produced in this step is referred to as High-boiling-point substance C.

In a process for industrially producing anone through a liquid phase oxidation of Cx by an oxygenous gas, High-boiling-point substance A and High-boiling-point substance B are usually brought into Step C to obtain a mixture of them with High-boiling-point substance C. A method of recovering anone and anol from such a high-boiling-point mixture includes a method of processing the mixture in two stages under a 5-100 atmosphere at 150-300 °C in the presence of an alkali, as proposed (Patent Document 1). However, this method has an industrially undesirable problem, because of the use of a large quantity of the alkali, which makes it difficult to transfer residues made highly viscous after the recovery of anone and anol. It is not easy to clean/remove the alkali from the residues and it is difficult to effectively utilize the residues as fuels.

A method for solving the problem includes a method of recovering products of anone and anol, which comprises adjusting an alkali concentration of 0.5 weight % or below by previous washing, and thermally decomposing a high-boiling-point mixture at 200-500 °C, as proposed (Patent Document 2). In this method, as the residues have fluidity and the content of the alkali is small, the residues can be utilized as fuels. The method uses a reaction distillation system, and accordingly requires a certain distillation time, which makes it difficult to downsize the reaction apparatus.

There is also a method of recovering products of anone and anol, which comprises thermally decomposing a by-product flow having a boiling point higher than anone, in the presence of an aluminum oxide catalyst under pressure at 200-450°C, as proposed (Patent Document 3). This method, however, newly requires a catalyst for decomposition of high-boiling-point substances as a reaction auxiliary. Further, such the contact decomposition surely requires a certain residence time, which makes it difficult to downsize the apparatus.
There is a further method of recovering products of anone and anol, which comprises thermally decomposing a by-product flow having a boiling point higher than anone, in the presence of a high aluminium oxide-containing catalyst (Patent Document 4). Also this method requires a catalyst as reaction auxiliary and a certain residence time.

Patent Document 1: JP 43-8266B
Patent Document 2: JP 8-81407A
Patent Document 3: JP 2001-139506A
Patent Document 4: US 6 255 533 B1

### DISCLOSURE OF THE INVENTION

### PROBLEM TO BE SOLVED BY THE INVENTION

The present invention has a subject to provide a method capable of efficiently recovering cyclohexanone and cyclohexanol from a high-boiling-point mixture produced as by-product in a process for producing cyclohexanone through a liquid phase oxidation of cyclohexane by an oxygenous gas, without the addition of a reaction auxiliary such as an alkali and a catalyst, even with the use of a small and simple reaction apparatus.

### MEANS TO SOLVE THE PROBLEM

The inventors et al. eagerly studied and consequently found that decomposition of the high-boiling-point mixture in the presence of subcritical or supercritical water makes it possible to efficiently recovery cyclohexanone and cyclohexanol, and finally reached completion of the invention.

The subject of the present invention can be solved by the following invention.
1) A method of recovering cyclohexanone and cyclohexanol, comprising: decomposing a high-boiling-point mixture in the presence of water at a temperature of 320 °C or higher under a pressure of 12 MPa or higher, the high-boiling-point mixture produced as by-product in a process for producing cyclohexanone through a liquid phase oxidation of cyclohexane by an oxygenous gas.
2) The method of recovering cyclohexanone and cyclohexanol according to the first invention, wherein the decomposing includes decomposition at the critical temperature of water or higher under the critical pressure of water or higher.
3) The method of recovering cyclohexanone and cyclohexanol according to the second invention, wherein the decomposing includes decomposition at a temperature of 600□ or lower under a pressure of 300 MPa or lower.
4) The method of recovering cyclohexanone and cyclohexanol according to the first invention, wherein the decomposing includes decomposition at a temperature lower than the critical temperature of water.
5) The method of recovering cyclohexanone and cyclohexanol according to the fourth invention, wherein the decomposing includes decomposition at a temperature of 330 °C or higher under a pressure of 12-300 MPa.
6) The method of recovering cyclohexanone and cyclohexanol according to the first invention, wherein the decomposing includes decomposition under a pressure lower than the critical pressure of water at the critical temperature of water or higher.
7) The method of recovering cyclohexanone and cyclohexanol according to the sixth invention, wherein the decomposing includes decomposition at a temperature of 600 °C or lower.
8) The method of recovering cyclohexanone and cyclohexanol according to the first invention, wherein the decomposing includes the use of a tube-type continuous flow reactor.

### EFFECT OF THE INVENTION

The present invention makes it possible to efficiently recover cyclohexanone and cyclohexanol from a high-boiling-point mixture produced as by-product in a process for producing cyclohexanone through a liquid phase oxidation of cyclohexane by an oxygenous gas, without the addition of a reaction auxiliary such as an alkali and a catalyst, even with the use of a small and simple reaction apparatus.

### THE BEST MODE FOR CARRYING OUT THE INVENTION

The present invention will now be described in detail.
The high-boiling-point mixture used in the present invention includes a mixture of substances having a higher boiling point than anone (High-boiling-point substances A, B, C), which are produced as by-product in each of the following steps in a process for producing cyclohexanone (hereinafter referred to as anone) through a liquid phase oxidation of cyclohexane (hereinafter referred to as Cx) by an oxygenous gas. The above high-boiling-point substances have been all publicly known in Patent Document 1 and the like.

1. High-boiling-point substance A: This is a high-boiling-point substance produced as by-product in the step of liquid-phase oxidizing Cx and the subsequent post-treatment step (referred to as Step A) such as the step of decomposing peroxides.
2. High-boiling-point substance B: This is a high-boiling-point substance produced as by-product in the step of dehydrogenating cyclohexanol (hereinafter referred to as anol) to invert it into anone (referred to as Step B).
3. High-boiling-point substance C: This is a high-boiling-point substance produced as by-product in the step of distillation purifying anone and anol (referred to as Step C).

Among the high-boiling-point substances, High-boiling-point substance A is contained 4-8 weight % in a solution, which mainly includes anone and anol obtained at Step A [for example, the step of processing a Cx oxidation reaction liquid with an aqueous alkali solution for saponification and for decomposition of cyclohexyl hydroperoxide, then separating and removing a water layer, and distilling and removing non-reacted Cx from the resultant oil layer]. The substance A comprises anol-esters, anone-condensed products, and other complicated condensed products.

High-boiling-point substance B is produced as by-product in a proportion of about 1 weight % to anone in Step B (the step of dehydrogenating anol) and consists of anone-condensed products and so forth. High-boiling-point substance C is produced as by-product in a proportion of about 1 weight % to the distillation material in Step C (the step of distilling and purifying anone and anol from the oil layer obtained in Step A, and the step of distilling and purifying anone obtained in Step B). The substance C consists of anol-esters and anone-condensed products produced through condensation, decomposition and recombination of anone and anol, and so forth.

In this way, High-boiling-point substance A and High-boiling-point substance B are generally brought into Step C. Accordingly, the high-boiling-point mixture of the present invention is obtained as a mixture of High-boiling-point substances A, B, C, that is, a distillation residue after distillation and purification of anone and anol for separation and recovery at Step C. By the present invention, it is possible to efficiently decompose the high-boiling-point mixture using the distillation residue as it is, without particularly executing an alkali treatment and water washing of the high-boiling-point mixture as is in the prior art. Because the high-boiling-point mixture is obtained as the distillation residue at Step C, it may contain a small amount of anone and anol and may further contain the alkali (such as a caustic alkali and an alkali metal carbonate) and a carboxylic acid. The contents thereof are not particularly restricted if they can fall within an inevitably accompanying range in obtaining the distillation residue.

In the present invention the high-boiling-point mixture is subjected to a decomposition reaction for decomposition in the presence of water on such a condition as, at a temperature of 320 °C or higher under a pressure of 12 MPa or higher. Preferably, it is achieved (1) at this temperature under this pressure and, at the same time, on such a condition as either: (2-1) at the critical temperature of water or higher under the critical pressure of water or higher; (2-2) at a temperature lower than the critical temperature of water; or (2-3) under a pressure lower than the critical pressure of water at the critical temperature of water or higher. A lower temperature than 320 °C results in insufficient decomposition of the high-boiling-point mixture undesirably. A lower pressure than 12 MPa similarly results in insufficient decomposition of the high-boiling-point mixture undesirably.

In the case of the conditions (1) and (2-1), the temperature can be not lower than the critical temperature of water (373.95 °C). An excessively higher temperature, though, may possibly result in a lowered yield because anone and anol are further subjected to reactions such as decomposition, and impose a severe environment on the apparatus material. Therefore, the temperature is not lower than the critical temperature of water (373.95 °C) and preferably 600 °C or lower, more preferably 500 °C or lower, and particularly preferably 450 °C or lower. The pressure can be not lower than the critical pressure of water (22.064 MPa). An excessively higher pressure, though, may require the use of a special material for the apparatus. Therefore, from the economic viewpoint, the pressure is preferably 300 MPa or lower, and more preferably 50 MPa or lower. In this case, water can be present 1 part by weight, preferably 10 parts by weight or more, relative to 100 parts by weight of the high-boiling-point mixture. The presence of a larger amount of water causes no problem on reactions and handling though less than 200 parts by weight is preferable to avoid energy consumption for heating water. There is no restriction on the density of water later described.

In the case of the conditions (1) and (2-2), the temperature can be not lower than 320 °C and lower than the critical temperature of water, preferably not lower than 330 °C and lower than the critical temperature of water. The pressure can be 12 MPa or higher, and preferably 15 MPa or higher. Similar to the above case, though, from the economic viewpoint, the pressure is preferably 300 MPa or lower, and more preferably 50 MPa or lower. Water can be controlled to exist such that the density of water is 0.05 kg/L or higher, more preferably 0.1 kg/L or higher, particularly preferably 0.1-0.8 kg/L, in a similar proportion to the high-boiling-point mixture, like in the case of the above conditions. The density of water is defined as the quotient derived from division of the supply of water (kg) by the space volume in a reactor (a value derived from subtraction of the volume of the high-boiling-point mixture from the reactor volume: unit L). The volume of the high-boiling-point mixture is herein a volume at the reaction temperature obtained on the assumption that the total amount of the mixture is anone (this is similarly applied in the following).

In the case of the conditions (1) and (2-3), the pressure can be not lower than 12 MPa and lower than the critical pressure of water, preferably not lower than 15 MPa and lower than the critical pressure of water. The temperature can be not lower than the critical temperature of water, similar to the case of the above conditions (1) and (2-1), not lower than the critical temperature of water (373.95 °C) and preferably 600 °C or lower, more preferably 500 °C or lower, and particularly preferably 450 °C or lower. The amount of water can be controlled to exist such that the density of water is 0.04 kg/L or higher, more preferably 0.06-0.73 kg/L, in a similar proportion to the high-boiling-point mixture, like in the case of the above conditions.

In the decomposition process of the present invention, the reaction system is not restricted, and any system, such as a batch type, a semi-batch type and a continuous flow type, may have no trouble if it can be used industrially in general. The reactor is not restricted particularly, and industrially available one in general, such as a tank type, a column type and a tube type, can be used appropriately. In the present invention, though, it is particularly preferable to use a small-diameter tube-type continuous flow reactor because of an extremely shorter decomposition time.

It is possible by the tube-type continuous flow reactor to achieve a substantial reaction time, for decomposition, of not less than 1 minute, and not more than 20 minutes, not more than 15 minutes, or not more than about 3 minutes. The substantial reaction time is the quotient derived from division of the reactor volume by the total amount of the high-boiling-point mixture and water at the reaction temperature. The volume of the high-boiling-point substance is the quotient derived from division of the supply of the high-boiling-point mixture (kg) by the density of the high-boiling-point mixture at the reaction temperature (kg/L; on the assumption that the total amount of the mixture is anol). The volume of water is the quotient derived from division of the supply of water (kg) by the above-described density of water (kg/L).

After the reaction, the reaction liquid is cooled for oil/water separation and then subjected to a normal distillation operation to recover anone and anol. On cooling, thermal recovery is performed preferably because the reaction liquid is still at a higher temperature. In the oil/water separation, the reaction liquid has a lower viscosity and the oil/water can be separated well. If required, a usual extraction solvent can be used without trouble, which includes an aliphatic hydrocarbon such as n-hexane, and cyclohexane; an aromatic hydrocarbon such as xylene, and toluene; and a ketone such as methylethyl ketone.

### EXAMPLES

The present invention will now be described specifically with Examples and Comparative examples. Anone and anol were analyzed by gas chromatography. The increments of anone and anol were represented by proportions (weight %) of the increased amounts of anone and anol to the material high-boiling-point mixture.

### [Example 1]

A reaction tube of stainless steel (an inner diameter of 0.5 mm and a length of 7.5 mm) was located in an electric oven. A tube-type continuous flow reactor, configured to quantitatively supply and mix the previously heated high-boiling-point mixture and water into the reaction tube through respective plunger pumps, was used for decomposition of the high-boiling-point mixture. The pressure is controlled with a back-pressure valve arranged at a piping outlet. The apparent residence time (Reactor volume (L)/Total supply of the high-boiling-point mixture and water (L/h); where the total supply is represented by a value at room temperature) is adjusted by the plunger pump discharge amount. The high-boiling-point mixture used was a high-boiling-point mixture (i) (a mixture of High-boiling-point substances A, B, C; with 11 weight % anol contained) obtained in a process for producing anone through a liquid phase oxidation of Cx by an oxygenous gas.

On condition that the reaction temperature is 350 °C, the reaction pressure is 30 MPa, the apparent residence time is 2.4 minutes and the supply ratio of the high-boiling-point mixture to water (Water/High-boiling-point mixture; weight basis) is 0.57, a reaction was performed for analysis. As a result, the increments of anone and anol were 11.0 weight % and 1.9 weight %, respectively. The density of water was calculated 0.63 kg/L from the reaction temperature, the reaction pressure and the supply ratio of the high-boiling-point mixture to water. The substantial reaction time was 1.4 minutes.

### [Example 2]

Except that the apparent residence time was changed to 1.5 minutes, a similar reaction was performed, as in Example 1. The density of water was 0.63 kg/L, and the substantial reaction time was 0.6 minutes. As a result, the increments of anone and anol were 9.1 weight % and 1.9 weight %, respectively.

### [Example 3]

Except that the reaction temperature was changed to 370 °C, a similar reaction was performed, as in Example 1. The density of water was 0.53 kg/L, and the substantial reaction time was 1.2 minutes. As a result, the increments of anone and anol were 12.1 weight % and 1.7 weight %, respectively.

### [Example 4]

Except that the reaction temperature was changed to 400 °C, and that the apparent residence time was changed to 1.1 minutes, a similar reaction was performed, as in Example 1. The density of water was 0.11 kg/L, and the substantial reaction time was 0.2 minute. As a result, the increments of anone and anol were 11.9 weight % and 1.7 weight %, respectively.

### [Example 5]

Except that the reaction pressure was changed to 20 MPa, and that the apparent residence time was changed to 1.2 minutes, a similar reaction was performed, as in Example 4. The density of water was 0.05 kg/L. As a result, the increments of anone and anol were 11.5 weight % and 1.6 weight %, respectively.

### [Example 6]

Except that the reaction pressure was changed to 40 MPa, a similar reaction was performed, as in Example 4. The density of water was 0.31 kg/L, and the substantial reaction time was 0.5 minutes. As a result, the increments of anone and anol were 11.6 weight % and 1.5 weight %, respectively.

### Comparative example 1]

Except that the reaction temperature was changed to 310 °C, and that the apparent residence time was changed to 1.1 minutes, a similar reaction was performed, as in Example 1. The density of water was 0.75 kg/L, and the substantial reaction time was 0.7 minute. As a result, the increments of anone and anol were 3.1 weight % and 1.6 weight %, respectively. The results from Examples 1-6 and Comparative example 1 are shown in Table 1.

**[Table 1]**

| | (1) | (2) | (3) | (4) | (5) | (6) | (7) | (8) |
|---|---|---|---|---|---|---|---|---|
| Example 1 | 350 | 30 | 2.4 | 1.4 | 0.63 | 11.0 | 1.9 | 12.9 |
| Example 2 | 350 | 30 | 1.5 | 0.6 | 0.63 | 9.1 | 1.9 | 11.0 |
| Example 3 | 370 | 30 | 2.4 | 1.2 | 0.53 | 12.1 | 1.7 | 13.8 |
| Example 4 | 400 | 30 | 1.1 | 0.2 | 0.11 | 11.9 | 1.7 | 13.6 |
| Example 5 | 400 | 20 | 1.2 | 0.1 | 0.05 | 11.5 | 1.6 | 13.1 |
| Example 6 | 400 | 40 | 1.1 | 0.5 | 0.31 | 11.6 | 1.5 | 13.0 |
| Comparative example 1 | 310 | 30 | 1.1 | 0.7 | 0.75 | 3.1 | 1.6 | 4.7 |

| | | | | | | | | |
|---|---|---|---|---|---|---|---|---|
| (1) Reaction Temperature (°C) (2) Reaction Pressure (MPa) (3) Apparent Residence Time (min) (4) Substantial Reaction Time (min) (5) Density of Water (kg/L) (6) Increment of Anone (%) (7) Increment of Anol (%) (8) Total Increment (%) | | | | | | | | |

### [Example 7]

Except that a reaction tube having an inner diameter of 3.2 mm and a length of 3.8 m was located, that a high-boiling-point mixture (ii) (containing 0.2 weight % anone, 11.0 weight % anol, and 4.5 weight % cyclohexenyl cyclohexanone) was used, and that 2158 g of the high-boiling-point mixture and 845 g of water were supplied on such a condition as, at a reaction temperature of 400 °C under a reaction pressure of 30 MPa for an apparent residence time of 2 minutes, a similar reactor was used to perform a reaction, as in Example 1. The resultant liquid was separated and a light liquid phase was distilled. As a result, 249 g of anone and 241 g of anol were obtained.

418 g of water was added to 1088 g of the distillation residue, and both were supplied to the reactor for a reaction on the above condition. As a result, 26.7 g of anone and 3.1 g of anol were obtained from the light liquid phase through distillation. 80 parts by weight of water were added to 100 parts by weight of the distillation residue, and both were fed into a pressure vessel for a further reaction on such a condition as, at a reaction temperature of 375 °C under a reaction pressure of 30 MPa for a reaction time of 40 minutes. As a result, 63.7 g of anone and 10.5 g of anol were obtained from the light liquid phase. On the other hand, the resultant heavy liquid phase (water phase) was found to contain 22.2 g of anone and 20.5 g of anol. Therefore, the amount of resultant anone in total was 361. 6 g, and the amount of resultant anol in total was 275.1 g. Thus, after subtraction of that in the material therefrom, the increment of anone was 357.3 g (an increase rate of 16.6 %), and the increment of anol was 38.4 g (an increase rate of 1.8 %).

### [Example 8]

Except that a high-boiling-point mixture (iii) (containing 11.7 weight % anol, 0.3 weight % cyclohexenyl cyclohexanone) was used, and that on such a condition as, at a reaction temperature of 370 °C under a reaction pressure of 30 MPa for an apparent residence time of 2.1 minutes at High-boiling-point mixture/Water (weight basis) of 2.5, a similar reactor was used to perform a reaction, as in Example 7. As a result, the resultant reaction solution was found to contain 12.1 weight % anone and 13.4 weight % anol.

### [Example 9]

3.00 g of the high-boiling-point mixture (i) and 3.00 g of water were added into a stainless-steel reaction tube (an inner diameter of 6 mm and a length of 35.4 cm) having one end sealed and the other end hermetically closed with a cap (connected to a temperature sensor and a pressure sensor) after replacement with nitrogen. The reaction tube was located in an electric oven and, after the inner temperature reached 350 °C (after 20 minutes elapsed), a reaction was performed for 10 minutes. The pressure during the reaction was 34 MPa and the density of water was calculated 0.64 kg/L. After the reaction, the reaction tube was thrown into cooled water for quenching, and isopropanol was added to the reacted product (separated into two layers of oil/water) to form a uniform phase, which was then analyzed. As a result, the increments of anone and anol were 13.1 weight % and 0.3 weight %, respectively.

### [Example 10]

Except that the high-boiling-point mixture was changed to 4.16 g and water to 0.13 g, a similar reaction was performed, as in Example 9. The reaction pressure was 15 MPa and the density of water was 0.05 kg/L. As a result, the increments of anone and anol were 7.4 weight % and 0.2 weight %, respectively.

### [Comparative example 2]

Except that the high-boiling-point mixture was changed to 5.21 g and water to 0.03 g, a similar reaction was performed, as in Example 9. The reaction pressure was 9 MPa and the density of water was 0.02 kg/L. As a result, the increments of anone and anol were merely 4.6 weight % and 0.1 weight %, respectively.

### [Comparative example 3]

Except that the high-boiling-point mixture was changed to 4.72 g and water to 0 g, a similar reaction was performed for analysis, as in Example 9. The reaction pressure was 4 MPa. As a result, the increment of anone was merely 4.4 weight %, and no anol could be recovered.

### [Example 11]

Except that the high-boiling-point mixture was changed to 2.61 kg and water to 2.61 g, and that the reaction temperature was changed to 380 °C, a similar reaction was performed, as in Example 9. The reaction pressure was 38 MPa and the density of water was 0.53 kg/L. As a result, the increments of anone and anol were 11.9 weight % and 0.6 weight %, respectively.

### [Example 12]

Except that the high-boiling-point mixture was changed to 1.04 g and water to 1.04 g, a similar reaction was performed, as in Example 10. The reaction pressure was 28 MPa and the density of water was 0.12 kg/L. As a result, the increments of anone and anol were 8.5 weight % and 0.2 weight %; respectively.

### [Comparative example 4]

Except that the high-boiling-point mixture was changed to 2.95 g and water to 0 g, a similar reaction was performed, as in Example 10. The reaction pressure was 7.6 MPa. As a result, the increment of anone was merely 3.8 weight % and anol was reduced by 2.7 weight %.

### [Example 13]

Except that the high-boiling-point mixture was changed to 2.09 g and water to 2.09 g, and that the reaction temperature was changed to 400 °C, a similar reaction was performed, as in Example 9. The reaction pressure was 41 MPa and the density of water was 0.36 kg/L. As a result, the increment of anone was 9.8 weight %. Anol was found to reduce by 0.2 weight %.

### [Example 14]

Except that the high-boiling-point mixture was changed to 0.88 g and water to 0.88 g, a similar reaction was performed, as in Example 13. The reaction pressure was 31 MPa and the density of water was 0.10 kg/L. As a result, the increment of anone was 9.2 weight %. Anol was found to reduce by 0.3 weight %.

### [Comparative example 5]

Except that the high-boiling-point mixture was changed to 2.33 g and water to 0 g, a similar reaction was performed, as in Example 13. The reaction pressure was 11 MPa. As a result, the increment of anone was merely 3.6 weight % and anol was found to reduce by 6.7 weight %. The results from Examples 9-14 and Comparative examples 2-5 are shown together in Table 2.

**[Table 2]**

| | (1) | (2) | (3) | (4) | (5) | (6) | (7) | (8) |
|---|---|---|---|---|---|---|---|---|
| Example 9 | 3.00 | 3.00 | 350 | 34 | 0.64 | 13.1 | 0.3 | 13.4 |
| Example 10 | 4.16 | 0.13 | 350 | 15 | 0.05 | 7.4 | 0.2 | 7.6 |
| Comparative e-example 2 | 5.21 | 0.03 | 350 | 9 | 0.02 | 4.6 | 0.1 | 4.7 |
| Comparative example 3 | 4.72 | 0 | 350 | 4 | - | 4.4 | 0 | 4.4 |
| Example 11 | 2.61 | 2.61 | 380 | 38 | 0.53 | 11.9 | 0.6 | 12.5 |
| Example 12 | 1.04 | 1.04 | 380 | 28 | 0.12 | 8.5 | 0.2 | 8.7 |
| Comparative example 4 | 2.95 | 0 | 380 | 7.6 | - | 3.8 | -2.7 | 1.1 |
| Example 13 | 2.09 | 2.09 | 400 | 41 | 0.36 | 9.8 | -0.2 | 9.6 |
| Example 14 | 0.88 | 0.88 | 400 | 31 | 0.10 | 9.2 | -0.3 | 8.9 |
| Comparative example 5 | 2.33 | 0 | 400 | 11 | - | 3.6 | -6.7 | -3.1 |

| | | | | | | | | |
|---|---|---|---|---|---|---|---|---|
| (1) High-boiling-point mixture (g) (2) Water (g) (3) Reaction Temperature (°C) (4) Reaction Pressure (MPa) (5) Density of Water (kg/L) (6) Increment of Anone (%) (7) Increment of Anol (%) (8) Total Increment (%) | | | | | | | | |

### INDUSTRIAL AVAILABILITY

The method of recovering cyclohexanone and cyclohexanol according to the present invention is capable of recovering cyclohexanone and cyclohexanol efficiently in a short time from a high-boiling-point mixture produced as by-product in a process for producing cyclohexanone through a liquid phase oxidation of cyclohexane by an oxygenous gas, without the addition of a reaction auxiliary such as an alkali and a catalyst, even with the use of a small and simple reaction apparatus, and thus it is extremely useful industrially.

## Claims

1. A method of recovering cyclohexanone and cyclohexanol, comprising:
decomposing a high-boiling-point mixture in the presence of water at a temperature of 320 °C or higher under a pressure of 12 MPa or higher, the high-boiling-point mixture produced as by-product in a process for producing cyclohexanone through a liquid phase oxidation of cyclohexane by an oxygenous gas.

2. The method of recovering cyclohexanone and cyclohexanol according to claim 1, wherein the decomposing includes decomposition at the critical temperature of water or higher under the critical pressure of water or higher.

3. The method of recovering cyclohexanone and cyclohexanol according to claim 2, wherein the decomposing includes decomposition at a temperature of 600 °C or lower under a pressure of 50 MPa or lower.

4. The method of recovering cyclohexanone and cyclohexanol according to claim 1, wherein the decomposing includes decomposition at a temperature lower than the critical temperature of water.

5. The method of recovering cyclohexanone and cyclohexanol according to claim 4, wherein the decomposing includes decomposition at a temperature of 330 °C or higher under a pressure of 12-300 MPa.

6. The method of recovering cyclohexanone and cyclohexanol according to claim 1, wherein the decomposing includes decomposition under a pressure lower than the critical pressure of water at the critical temperature of water or higher.

7. The method of recovering cyclohexanone and cyclohexanol according to claim 6, wherein the decomposing includes decomposition at a temperature of 600 °C or lower.

8. The method of recovering cyclohexanone and cyclohexanol according to claim 1, wherein the decomposing includes the use of a tube-type continuous flow reactor.

## Patentansprüche

1. Verfahren zum Rückgewinnen von Cyclohexanon und Cyclohexanol, umfassend:
Zersetzen eines Gemischs mit hohem Siedepunkt in der Gegenwart von Wasser bei einer Temperatur von 320°C oder höher unter einem Druck von 12 MPa oder höher, wobei das Gemisch mit hohem Siedepunkt als Nebenprodukt in einem Verfahren zur Herstellung von Cyclohexanon durch Flüssigphasenoxidation von Cyclohexan durch ein sauerstofffaltiges Gas hergestellt wird.

2. Verfahren zum Rückgewinnen von Cyclohexanon und Cyclohexanol nach Anspruch 1, wobei das Zersetzen die Zersetzung bei der kritischen Wassertemperatur oder höher unter dem kritischen Wasserdruck oder höher einschließt.

3. Verfahren zum Rückgewinnen von Cyclohexanon und Cyclohexanol nach Anspruch 2, wobei das Zersetzen die Zersetzung bei einer Temperatur von 600°C oder niedriger unter einem Druck von 50 MPa oder niedriger einschließt.

4. Verfahren zum Rückgewinnen von Cyclohexanon und Cyclohexanol nach Anspruch 1, wobei das Zersetzen die Zersetzung bei einer niedrigeren Temperatur als die kritische Wassertemperatur einschließt.

5. Verfahren zum Rückgewinnen von Cyclohexanon und Cyclohexanol nach Anspruch 4, wobei das Zersetzen die Zersetzung bei einer Temperatur von 330°C oder höher unter einem Druck von 12-300 MPa einschließt.

6. Verfahren zum Rückgewinnen von Cyclohexanon und Cyclohexanol nach Anspruch 1, wobei das Zersetzen die Zersetzung unter einem niedrigeren Druck als der kritische Wasserdruck bei der kritischen Wassertemperatur oder höher einschließt.

7. Verfahren zum Rückgewinnen von Cyclohexanon und Cyclohexanol nach Anspruch 6, wobei das Zersetzen die Zersetzung bei einer Temperatur von 600°C oder niedriger einschließt.

8. Verfahren zum Rückgewinnen von Cyclohexanon und Cyclohexanol nach Anspruch 1, wobei das Zersetzen die Verwendung eines röhrenförmigen Reaktors mit kontinuierlichem Fluss einschließt.

## Revendications

1. Un procédé de récupération de la cyclohexanone et du cyclohexanol, comprenant :
la décomposition d'un mélange à point d'ébullition élevé en présence d'eau à une température de 320°C ou supérieure sous une pression de 12 MPa ou supérieure, le mélange à point d'ébullition élevé étant produit en tant que sous-produit dans un processus de production de cyclohexanone par l'intermédiaire d'une oxydation en phase liquide du cyclohexane par un gaz oxygéné.

2. Le procédé de récupération de la cyclohexanone et du cyclohexanol de la revendication 1, dans lequel la décomposition inclut la décomposition à la température critique de l'eau ou supérieure sous la pression critique de l'eau ou supérieure.

3. Le procédé de récupération de la cyclohexanone et du cyclohexanol de la revendication 2, dans lequel la décomposition inclut la décomposition à une température de 600°C ou inférieure sous une pression de 50 MPa ou inférieure.

4. Le procédé de récupération de la cyclohexanone et du cyclohexanol de la revendication 1, dans lequel la décomposition inclut la décomposition à une température inférieure à la température critique de l'eau.

5. Le procédé de récupération de la cyclohexanone et du cyclohexanol de la revendication 4, dans lequel la décomposition inclut la décomposition à une température de 330°C ou supérieure sous une pression de 12 à 300 MPa.

6. Le procédé de récupération de la cyclohexanone et du cyclohexanol de la revendication 1, dans lequel la décomposition inclut la décomposition sous une pression inférieure à la pression critique de l'eau à la température critique de l'eau ou supérieure.

7. Le procédé de récupération de la cyclohexanone et du cyclohexanol de la revendication 6, dans lequel la décomposition inclut la décomposition à une température de 600°C ou inférieure.

8. Le procédé de récupération de la cyclohexanone et du cyclohexanol de la revendication 1, dans lequel la décomposition comprend l'utilisation d'un réacteur à flux continu de type tubulaire.
